# EUROPEAN PATENT APPLICATION

(11) **EP 4 049 678 A1**
(43) Date of publication of application: **31.08.2022**
(21) Application number: 20878639.2
(22) Date of filing: 23.10.2020
(51) Int. Cl.: A61K 45/00, A61P 1/02, A61P 43/00, A61K 6/889, A61K 31/231

(54) **AGENT FOR PROMOTING ODONTOBLAST PROPAGATION AND DIFFERENTIATION**

(30) Priority: 23.10.2019 JP 2019193019
(71) Applicant: Sun Medical Co., Ltd., Shiga 524-0044 (JP)
(72) Inventor: SAITO, Takashi, Ebetsu-shi, Hokkaido 067-0004 (JP); QIU, Youjing, Ishikarigun, Hokkaido 0610223 (JP); OTSUKI, Tamaki, Moriyama-shi, Shiga 524-0044 (JP); TADA, Akiyo, Moriyama-shi, Shiga 524-0044 (JP); YAMAMOTO, Takashi, Moriyama-shi, Shiga 524-0044 (JP); ORI, Tatsuya, Moriyama-shi, Shiga 524-0044 (JP); NAKAMURA, Kumiko, Moriyama-shi, Shiga 524-0044 (JP); ISHIDA, Tadashi, Moriyama-shi, Shiga 524-0044 (JP)
(74) Representative: Hoffmann Eitle
(86) International application number: PCT/JP2020/039911
(87) International publication number: WO 2021/079988

(57) **Abstract**

An odontoblast proliferation/differentiation promoting agent contains a calcium salt of 4- (meth) acryloyloxyethyltrimellitic acid.

## Description

### TECHNICAL FIELD

The present invention relates to an odontoblast proliferation/differentiation promoting agent.

### BACKGROUND ART

In dental treatment of dental caries (bad tooth), there has been known that a dental caries portion is removed and then a restorative material such as a crown, an inlay, or a composite resin is adhered to the tooth by a dental adhesive. For example, such dental adhesive is applied after a tooth substance (dentin) is decalcified, and the applied adhesive is penetrated into the decalcified portion, to thereby form a hybrid layer. Thus, the dental adhesive improves adhesiveness between the restorative material and the tooth structure.

When a caries infection site reaches deep within the tooth structure, it may reach a dental pulp upon removal of the caries, which may cause sudden pulp exposure around the infection site. Therefore, such dental adhesive is liable to reduce the strength of the tooth structure or adversely affect the dental pulp (nerve) . For this reason, various studies have been made to add a dental material having a recalcification ability of a tooth structure to the dental adhesive

There has been proposed, for example, a dental material containing a monomer calcium salt which is formed by neutralizing an acidic group of a monomer having a polymerizable group and an acidic group with calcium and/or a polymer calcium salt which is a polymer of a monomer having a polymerizable group and an acidic group and is formed by neutralizing the acidic group with calcium (see, for example, Patent Literature 1).

The recalcification ability of the dental material is confirmed by adding calcium chloride, potassium dihydrogen phosphate, potassium chloride, and sodium azide to a Hepes aqueous solution to prepare a calcification solvent, adding the above dental material to the calcification solvent, and then measuring a precipitated amount of calcium produced in the calcification solvent.

### CITATION LIST

### PATENT DOCUMENT

Patent Document 1: JP 2006-347943 A

### SUMMARY OF INVENTION

### PROBLEMS TO BE SOLVED BY THE INVENTION

When dental caries is close to or reaches a dental pulp, dentin around the dental pulp or a portion of the dental pulp that has been infected by pathogenic bacteria of dental caries needs to be removed. In recent years, complete removal of a dental pulp (pulpectomy) has caused weakening of a tooth structure, particularly dentin. Accordingly, while an infected portion of the dental pulp is removed, an uninfected portion thereof is desired to be preserved.

In this case, removal of the infected portion of the dental pulp provides a space in a dental pulp cavity, to thereby expose the uninfected portion thereof. Therefore, it is considered that the dental pulp is preserved in a vital state by filling the space in the dental pulp cavity with a pulp capping agent to cover the dental pulp.

For example, an indirect pulp capping method has been proposed such that a calcium hydroxide-based medical agent is used as a pulp capping agent, and the calcium hydroxide stimulates odontoblasts to promote formation of a dentin bridge in a portion of the dental pulp contacted with the medical agent. Such indirect pulp capping method allows a portion of a vital pulp to differentiate into dentin, so that the dental pulp itself is reduced in volume, which may cause atrophy of the dental pulp

As described above, the dental material described in Patent Literature 1 is usually added to a dental adhesive, not to a pulp capping agent.

The present invention provides an odontoblast proliferation/differentiation promoting agent capable of promoting proliferation of an odontoblast and/or differentiation of a stem cell into an odontoblast.

### MEANS FOR SOLVING PROBLEMS

The present invention [1] includes an odontoblast proliferation/differentiation promoting agent containing a calcium salt of 4-(meth)acryloyloxyethyltrimellitic acid.

The present invention [2] includes the odontoblast proliferation/differentiation promoting agent according to [1] above, further containing a bioabsorbable material that dissolves, disperses, or adsorbs the calcium salt of 4-(meth)acryloyloxyethyltrimellitic acid.

The present invention [3] includes the odontoblast proliferation/differentiation promoting agent described in [1] or [2] above, further containing an anti-inflammatory agent.

### EFFECTS OF THE INVENTION

Since the odontoblast proliferation/differentiation promoting agent of the present invention contains a calcium salt of 4-(meth)acryloyloxyethyltrimellitic acid, proliferation of odontoblasts and/or differentiation of stem cells into odontoblasts can be promoted. Therefore, in dental treatment, by bringing the odontoblast proliferation/differentiation promoting agent into contact with a dental pulp exposed when an infected portion of the dental pulp is removed, odontoblasts can be expected to proliferate in a space in a dental pulp cavity. In this case, proliferated odontoblasts form dentin, so that a volume reduction of the dental pulp cavity can be suppressed, which in turn can prevent atrophy of the dental pulp.

### BRIEF DESCRIPTION OF DRAWINGS

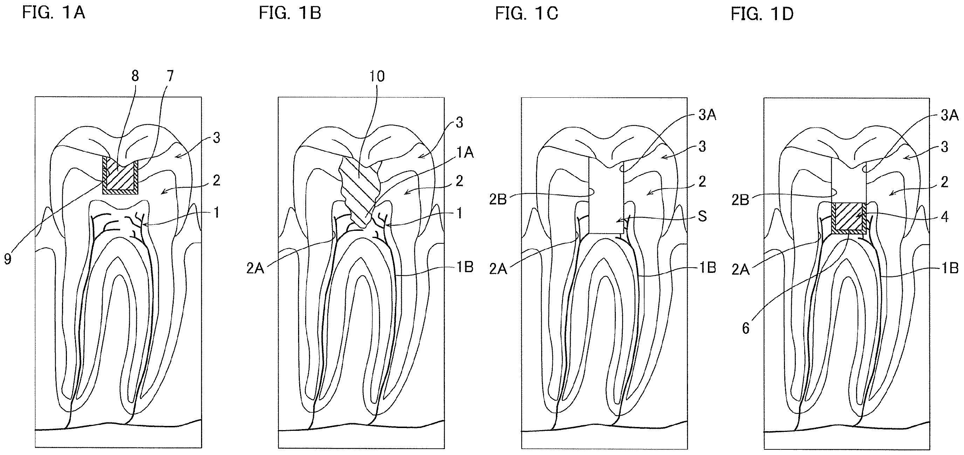

FIG.1A shows a schematic configuration diagram of a tooth in which dental caries has not yet reached a dental pulp. FIG. 1B shows a schematic configuration diagram of a tooth in which dental caries has reached a dental pulp. FIG. 1C, subsequent to FIG. 1B, shows a schematic configuration diagram of the tooth in which the dental pulp is exposed by removing an infected portion thereof. FIG. 1D, subsequent to FIG. 1C, shows a schematic configuration diagram of the tooth in which the exposed dental pulp is covered with a conventional pulp capping agent.

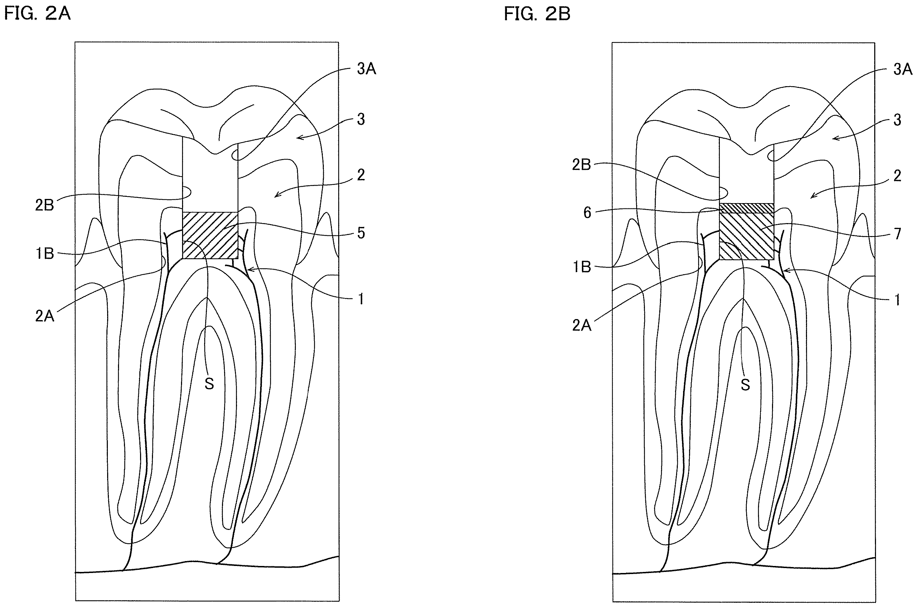

FIG. 2A, subsequent to FIG. 1C, shows a schematic configuration diagram of the tooth in which the exposed dental pulp is covered with an odontoblast proliferation/differentiation promoting agent. FIG. 2B, subsequent to FIG. 2A, shows a schematic configuration diagram of the tooth in a state where odontoblasts proliferate and differentiate so that a dental pulp cavity is filled.

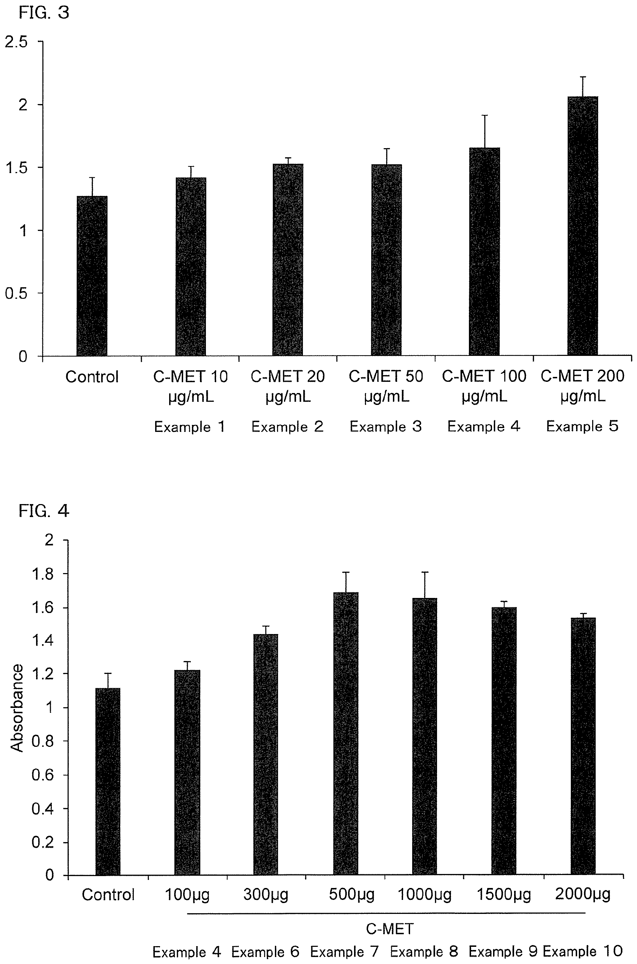

FIG. 3 is a graph showing the results of a CCK-8 assay of rat odontoblast-like cell lines allowed to proliferate with the odontoblast proliferation/differentiation promoting agents of Examples 1 to 5.
FIG. 4 is a graph showing the results of a CCK-8 assay of rat odontoblast-like cell lines allowed to proliferate with the odontoblast proliferation/differentiation promoting agents of Examples 4, 6 to 10.

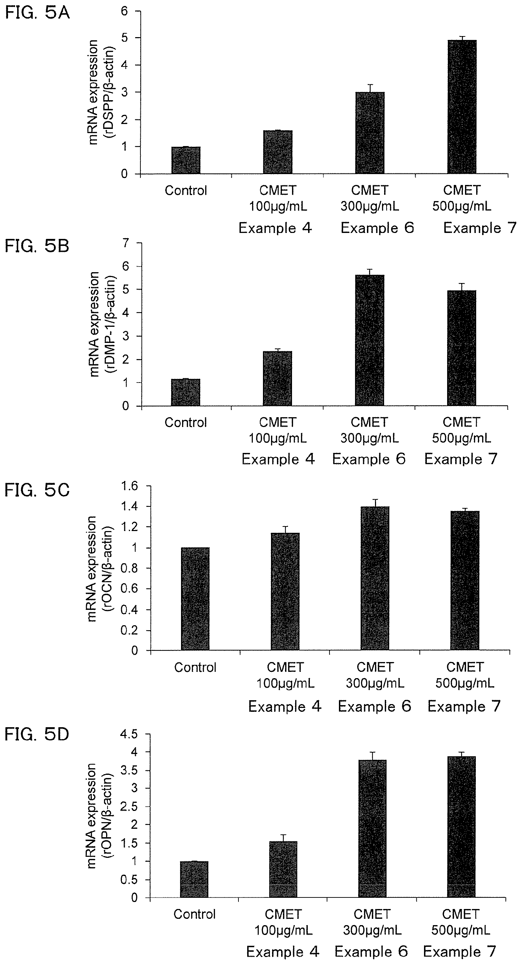

FIG. 5A shows real-time PCR analysis results of an odontoblast proliferation test using the odontoblast proliferation/differentiation promoting agents of Examples 4, 6 and 7, and shows mRNA expression levels of rDSPP. FIG. 5B shows real-time PCR analysis results of the same odontoblast proliferation test as in FIG. 5A and shows mRNA expression levels of rDMP-1. FIG. 5C shows real-time PCR analysis results of the same odontoblast proliferation test as in FIG. 5A and shows mRNA expression levels of rOCN. FIG. 5D shows real-time PCR analysis results of the same odontoblast proliferation test as in FIG. 5A and shows expression levels of rOPN.

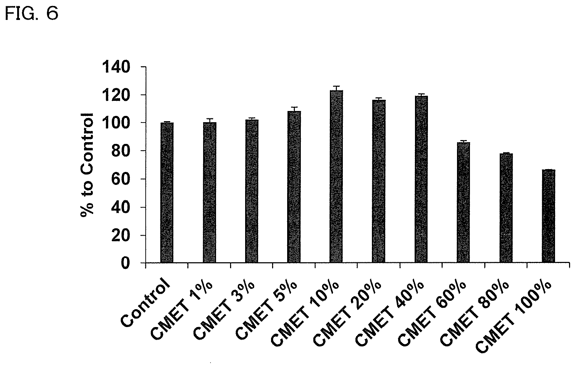

FIG. 6 is a graph showing the results of a CCK-8 assay of human dental pulp stem cells that have been contacted with the odontoblast proliferation/differentiation promoting agents of Examples 9, 11 to 17.

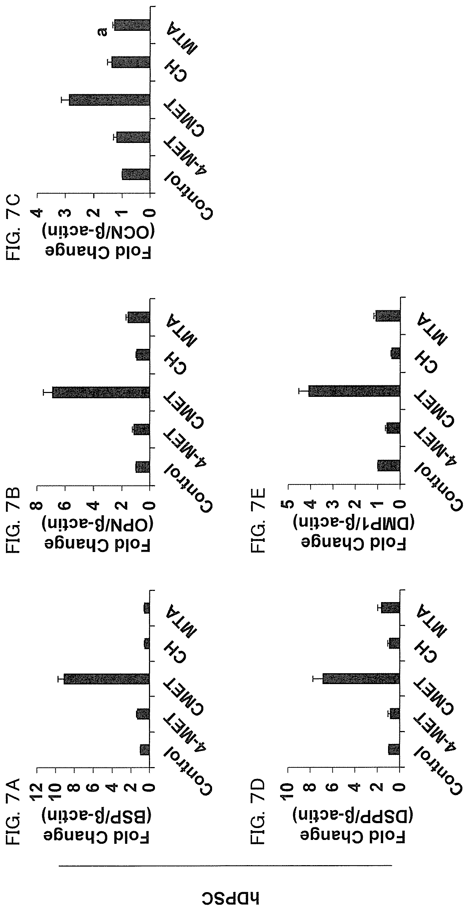

FIG. 7A shows real-time PCR analysis results of a differentiation test of human dental pulp stem cells into odontoblasts using the odontoblast proliferation/differentiation promoting agents of Example 12 and Comparative Examples 1 to 3, and shows mRNA expression levels of BSP. FIG. 7B shows real-time PCR analysis results of the same differentiation test of human dental pulp stem cells into odontoblasts as in FIG. 7A and shows mRNA expression levels of rOPN. FIG. 7C shows real-time PCR analysis results of the same differentiation test of human dental pulp stem cells into odontoblasts as in FIG. 7A and shows mRNA expression levels of rOCN. FIG. 7D shows real-time PCR analysis results of the same differentiation test of human dental pulp stem cells into odontoblasts as in FIG. 7A and shows mRNA expression levels of rDSPP. FIG. 7E shows real-time PCR analysis results of the same differentiation test of human dental pulp stem cells into odontoblasts as in FIG. 7A and shows mRNA expression levels of rDMP-1.

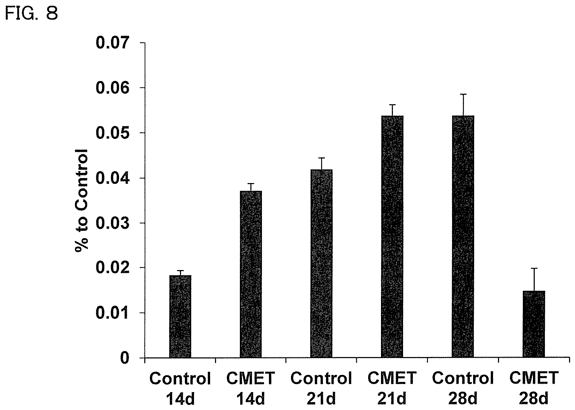

FIG. 8 is a graph showing the results of alkaline phosphatase measurement (standing times for 14, 21, and 28 days) using the odontoblast proliferation/differentiation promoting agent of Example 12.

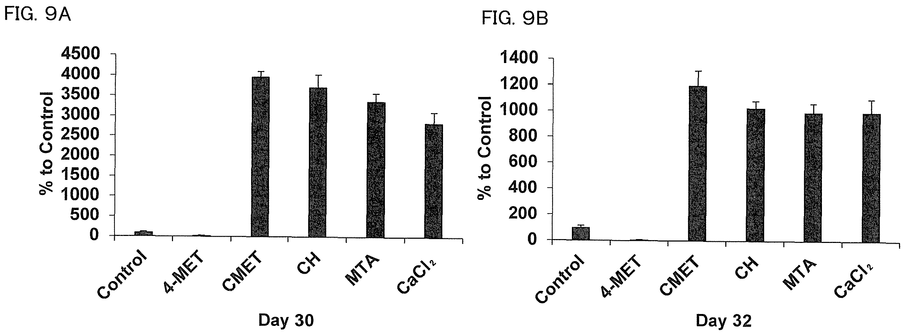

FIG. 9A is a graph showing the results of alizarin red S staining measurement (standing time for 30 days) using the odontoblast proliferation/differentiation promoting agents of Example 12 and Comparative Examples 1 to 4. FIG. 9B is a graph showing the results of the same alizarin red S staining measurement (standing time for 32 days) as in FIG. 9A.

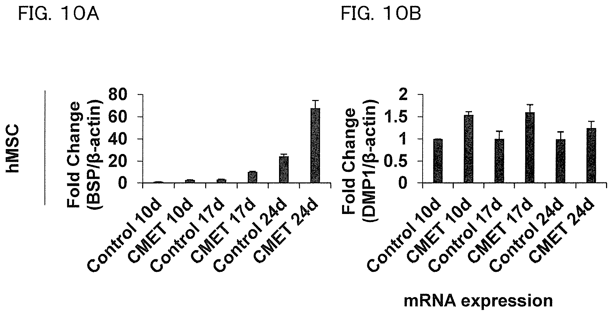

FIG. 10A shows real-time PCR analysis results of a differentiation test of mesenchymal stem cells into odontoblasts using the odontoblast proliferation/differentiation promoting agent of Example 12 and shows mRNA expression levels of BSP. FIG. 10B shows real-time PCR analysis results of the same differentiation test of mesenchymal stem cells into odontoblasts as in FIG. 10A and shows mRNA expression levels of rDMP-1.

### MODES FOR CARRYING OUT THE INVENTION

The odontoblast proliferation/differentiation promoting agent of the present invention contains a calcium salt of 4-(meth)acryloyloxyethyltrimellitic acid as an essential component.

### (1) Calcium salt of 4-(meth)acryloyloxyethyltrimellitic acid

The calcium salt of 4-(meth)acryloyloxyethyltrimellitic acid is represented by the following general formula (1): 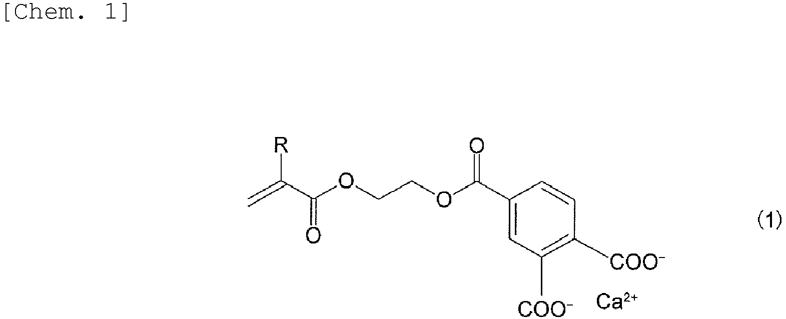 where R is H or CH₃.

In general formula (1), R is a hydrogen atom or a methyl group, preferably a methyl group.

That is, the odontoblast proliferation/differentiation promoting agent contains a calcium salt of 4-acryloyloxyethyltrimellitic acid and/or a calcium salt of 4-methacryloyloxyethyltrimellitic acid (hereinafter referred to as C-MET), preferably C-MET.

When the odontoblast proliferation/differentiation promoting agent has a solid content of 100% by mass, it contains the calcium salt of 4- (meth) acryloyloxyethyltrimellitic acid in a proportion of, for example, 0.01% by mass or more, preferably 0.03% by mass or more, and for example, 100% by mass or less, preferably 95% by mass or less.

When the content of the calcium salt of 4-(meth) acryloyloxyethyltrimellitic acid is the lower limit or more, proliferation of odontoblasts and/or differentiation of stem cells into odontoblasts can be securely promoted. When the content of the calcium salt of 4-(meth)acryloyloxyethyltrimellitic acid is the upper limit or less, the effect of promoting proliferation of odontoblasts and/or differentiation of stem cells into odontoblasts can be secured and the amount of the calcium salt of 4-(meth)acryloyloxyethyltrimellitic acid used can be reduced.

### (2) Bioabsorbable material

The odontoblast proliferation/differentiation promoting agent preferably further contains a bioabsorbable material as an optional component.

If the odontoblast proliferation/differentiation promoting agent contains a bioabsorbable material, when the odontoblast proliferation/differentiation promoting agent is brought into contact with a dental pulp, a sufficient space for proliferation of odontoblasts and/or differentiation of stem cells into odontoblasts can be secured (see FIG. 2A).

The bioabsorbable material dissolves, disperses, or adsorbs the calcium salt of 4-(meth)acryloyloxyethyltrimellitic acid.

The bioabsorbable material is a material that can be absorbed by a living body, and examples thereof include collagen (e.g., type I collagen, type II collagen, type III collagen, atelocollagen, etc.), proteins (e.g., gelatin, fibrin, etc.), polysaccharides (e.g., oxycellulose, chitin, chitosan, hyaluronic acid, etc.), synthetic polymers (e.g., polyglycolic acid copolymer, polylactic acid copolymer, etc.), and phosphoric acid-based and carbonic acid-based inorganic materials (e.g., hydroxyapatite, tricalcium phosphate, calcium carbonate, etc.). The bioabsorbable material can be used alone or in combination of two or more kinds.

Of the bioabsorbable materials, preferably, collagen is used.

A state of the bioabsorbable material at ordinary temperature (25°C) is not particularly limited and may be a liquid or a solid. When in a liquid state at ordinary temperature, the bioabsorbable material dissolves or disperses the calcium salt of 4-(meth)acryloyloxyethyltrimellitic acid. When in a solid state at ordinary temperature, the bioabsorbable material adsorbs the calcium salt of 4-(meth)acryloyloxyethyltrimellitic acid.

The content ratio of the bioabsorbable material with respect to 1 part by mass of 4-(meth)acryloyloxyethyltrimellitic acid is , for example, 2 parts by mass or more, preferably 5 parts by mass or more, and for example, 100 parts by mass or less, preferably 80 parts by mass or less.

### (3) Anti-inflammatory agent

The odontoblast proliferation/differentiation promoting agent preferably further contains an anti-inflammatory agent as an optional component.

If the anti-inflammatory agent contains a bioabsorbable material, when the odontoblast proliferation/differentiation promoting agent is in contact with a dental pulp, inflammation of the dental pulp can be suppressed and proliferation of odontoblasts and/or differentiation of stem cells into odontoblasts can also be securely promoted (see FIG. 2A).

The anti-inflammatory agent is not particularly limited as long as it is a known anti-inflammatory agent applicable to dental materials, and examples thereof include steroidal anti-inflammatory agents (e.g., dexamethasone, prednisolone, beclomethasone, etc.), betamethasone, fluticasone, and hydrocortisone. The anti-inflammatory agent can be used alone or in combination of two or more kinds.

Of the anti-inflammatory agents, preferably, a steroidal anti-inflammatory agent is used, more preferably, dexamethasone is used.

The content ratio of the anti-inflammatory agent with respect to 1 part by mass of 4-(meth)acryloyloxyethyltrimellitic acid is , for example, 1 × 10⁻⁷ parts by mass or more, preferably 1 × 10⁻⁶ parts by mass or more, and for example, 1 × 10⁻² parts by mass or less, preferably 1 × 10⁻³ parts by mass or less.

### (4) Additional additives

Further, the odontoblast proliferation/differentiation promoting agent can contain additional additives as optional components at appropriate ratios. Examples of additional additives include excipients, preservatives, buffering agents, coloring agents, flavoring agents, and thickening agents.

### (5) Formulation of odontoblast proliferation/differentiation promoting agent

The above-mentioned odontoblast proliferation/differentiation promoting agent can be suitably used as a pulp capping agent, for example. When dental caries reaches a dental pulp, the odontoblast proliferation/differentiation promoting agent is used so as to be brought into contact with the dental pulp exposed by dental treatment (see FIG. 2A). The formulation of the odontoblast proliferation/differentiation promoting agent is not particularly limited, and examples thereof include a powder formulation, a liquid formulation, and a sheet formulation.

### (5-1) Powder formulation

When the odontoblast proliferation/differentiation promoting agent is in a powder formulation, the calcium salt of 4-(meth)acryloyloxyethyltrimellitic acid preferably has a particle shape.

The calcium salt of 4-(meth)acryloyloxyethyltrimellitic acid has an average primary particle size of, for example, 1 µm or more, preferably 5 µm or more, and for example, 100 µm or less, preferably 50 µm or less.

To use the odontoblast proliferation/differentiation promoting agent in a powder formulation, for example, the odontoblast proliferation/differentiation promoting agent is once retained in a cotton ball, and the cotton ball is then brought into contact with or swung near a dental pulp to fix the odontoblast proliferation/differentiation promoting agent to the dental pulp (see FIG. 2A).

### (5-2) Liquid formulation

When the odontoblast proliferation/differentiation promoting agent is in a liquid formulation, the odontoblast proliferation/differentiation promoting agent preferably contains a solvent.

The solvent can dissolve or disperse the calcium salt of 4-(meth)acryloyloxyethyltrimellitic acid.

Examples of the solvent include high polar solvents (e.g., water, ethanol, etc.), medium polar solvents (e.g., acetone, etc.), and low polar solvents (e.g., oil, etc.). The solvent can be used alone or in combination of two or more kinds.

Of the solvents, preferably, water, ethanol, and acetone are used.

The concentration of the calcium salt of 4-(meth)acryloyloxyethyltrimellitic acid in the odontoblast proliferation/differentiation promoting agent in a liquid formulation is, for example, 1 µg/mL or more, preferably 10 µg/mL or more, more preferably 150 µg/mL or more, further more preferably 300 µg/mL or more, and for example, 5000 µg/mL or less, preferably 3000 µg/mL or less, more preferably 2000 µg/mL or less, particularly preferably 1200 µg/mL or less.

When the concentration of the calcium salt of 4-(meth)acryloyloxyethyltrimellitic acid is the lower limit or more, proliferation of odontoblasts and/or differentiation of stem cells into odontoblasts can be more securely promoted. When the concentration of the calcium salt of 4-(meth)acryloyloxyethyltrimellitic acid is the upper limit or less, the effect of promoting proliferation of odontoblasts and/or differentiation of stem cells into odontoblasts can be sufficiently secured and the amount of the calcium salt of 4-(meth)acryloyloxyethyltrimellitic acid used can be reduced.

To use the odontoblast proliferation/differentiation promoting agent in a liquid formulation, for example, the odontoblast proliferation/differentiation promoting agent is applied, sprayed, or added dropwise to a dental pulp (see FIG. 2A).

### (5-3) Sheet formulation

When the odontoblast proliferation/differentiation promoting agent is in a sheet formulation, the calcium salt of 4-(meth)acryloyloxyethyltrimellitic acid is preferably adsorbed to a sheet formed of a bioabsorbable material (e.g., collagen sheet, etc.).

In this case, the calcium salt of 4-(meth)acryloyloxyethyltrimellitic acid preferably has a particle shape. The average primary particle size of the calcium salt of 4-(meth)acryloyloxyethyltrimellitic acid is, for example, in the same range as the above-mentioned range.

To use the odontoblast proliferation/differentiation promoting agent in a sheet formulation, for example, the odontoblast proliferation/differentiation promoting agent is stuck to a tooth so that the calcium salt of 4- (meth) acryloyloxyethyltrimellitic acid comes into contact with a dental pulp (see FIG. 2A).

### (5) Effects

When dental caries does not reach a dental pulp 1, in dental treatment, a caries portion of an enamel 3 and a primary dentin 2 is removed to form, for example, a cavity 9, as shown in FIG. 1A. An inner surface of the cavity 9 is decalcified. Thereafter, a known dental adhesive 7 is applied to the inner surface of the cavity 9, and a restorative material 8 (e.g., an inlay, a composite resin, etc.) is adhered to the tooth with the dental adhesive 7. That is, when the dental caries does not reach the dental pulp 1, the dental adhesive 7 is used so as to come into contact with surfaces of the enamel 3 and the primary dentin 2 and so as not to come into contact with the dental pulp 1.

On the other hand, as shown in FIG. 1B, when dental caries 10 reaches the dental pulp 1, in dental treatment, an infected portion 1A of the dental pulp 1 that has been infected by pathogenic bacteria of dental caries is removed as well as the caries portion of the enamel 3 and the primary dentin 2, and an uninfected portion 1B of the dental pulp is preserved. In this case, as shown in FIG. 1C, the removal of the infected portion 1A provides a space S in a dental pulp cavity 2A that the primary dentin 2 has, and a part of the uninfected portion 1B is exposed through a cavity 3A formed in the enamel 3, a cavity 2B formed in the primary dentin 2, and the space S.

As shown in FIG. 1D, when a conventional pulp capping agent 4 (e.g., a calcium hydroxide-based medical agent) is brought into contact with the uninfected portion 1B thus exposed, odontoblasts included in the uninfected portion 1B are stimulated to form tertiary dentin 6 only on the surface of the uninfected portion 1B facing the space S in the dental pulp cavity 2A. Therefore, the dental pulp cavity 2A is reduced in volume, which may cause atrophy of the dental pulp 1.

On the other hand, as shown in FIG. 2A, when an odontoblast proliferation/differentiation promoting agent 5 containing a calcium salt of 4-(meth)acryloyloxyethyltrimellitic acid is brought into contact with the uninfected portion 1B thus exposed, as shown in FIG. 2B, proliferation of odontoblasts included in the uninfected portion 1B and/or differentiation of stem cells into odontoblasts can be promoted, and the odontoblasts 7 can be expected to proliferate so as to fill the space S as well as the surface of the uninfected portion 1B facing the space S in the dental pulp cavity 2A. Then, the proliferated odontoblasts form the tertiary dentin 6 on the outside of the dental pulp cavity 2A (e.g., in the cavity 2B of the primary dentin 2), so that volume reduction of the dental pulp cavity 2A can be suppressed, which in turn can prevent atrophy of the dental pulp 1.

### EXAMPLES

Hereinafter, the present invention will be described more in detail with reference to examples, but not limited to the examples. Specific numerical values such as blending ratios (content), physical property values, and parameters used in the following description can be replaced with the upper limit value (numeral values defined with "or less" or "less than") or the lower limit value (numeral values defined with "or more" or "more than") of the corresponding blending ratios (content), physical property values, and parameters described in the above-mentioned "MODES FOR CARRYING OUT THE INVENTION".

### <Examples 1 to 10>

### <<Preparation of calcium salt of 4-methacryloyloxyethyltrimellitic acid>>

To a glass reactor equipped with a stirrer, a condenser tube, a thermometer, and a dropping funnel were added 64 g of ethanol and 3.19 g (0.01 mol) of 4-methacryloxyethyltrimellitic acid (hereinafter referred to as 4-MET), and the mixture was stirred while an internal temperature of the reactor was cooled to about 10°C with ice water, to thereby dissolve 4-MET in the ethanol.

Separately, 0.74 g (0.01 mol) of calcium hydroxide was dissolved in 400 g of distilled water to prepare an aqueous calcium hydroxide solution. Then, the aqueous calcium hydroxide solution was added dropwise to the ethanol solution of 4-MET with the dropping funnel for about 30 minutes. At this time, the internal temperature of the reactor was kept unchanged.

For 30 minutes after completion of the dropping of the ethanol solution of 4-MET, the internal temperature of the reactor was increased to room temperature (25°C) and stirring of the reaction mixture was continued to age the reaction product of 4-MET and calcium hydroxide. The reaction mixture thus obtained was concentrated in a vacuum distiller and the precipitated reaction product was filtered. The residue was washed with ethanol and then dried to give about 3.3 g of a white solid powder.

The solid powder thus obtained was confirmed to be a calcium salt of 4-MET (C-MET) by NMR analysis and elemental analysis. An acid-base gram equivalent ratio (calcium base gram equivalent/acid gram equivalent) of the C-MET was 1.0 [gram equivalent/gram equivalent].

C-MET was roughly pulverized with a ball mill and the pulverized product was further sufficiently pulverized to a uniform fine powder in an agate mortar. When a portion of the C-MET powder was observed with a scanning electron micrograph, the C-MET powder had a maximum primary particle size of about 30 µm and a minimum average primary particle size of about 0.1 µm. The C-MET powder was subjected to gas sterilization with ethylene oxide, packaged, and then stored.

### <<Preparation of odontoblast proliferation/differentiation promoting agent>>

Next, the C-MET powder was dissolved or dispersed in ultrapure water (dH₂O) in order to make the following concentrations of C-MET in Examples, to thereby prepare an odontoblast proliferation/differentiation promoting agent in a liquid formulation. In Example 17, C-MET was not dissolved or dispersed in ultrapure water, and powdery C-MET was determined as an odontoblast proliferation/differentiation promoting agent in a powder formulation.
Example 1: 10 µg/mL (1.0% by mass)
Example 2: 20 µg/mL (2.0% by mass)
Example 3: 50 µg/mL (4.8% by mass)
Example 4: 100 µg/mL (9.1% by mass)
Example 5: 200 µg/mL (16.7% by mass)
Example 6: 300 µg/mL (23.1% by mass)
Example 7: 500 µg/mL (33.3% by mass)
Example 8: 1000 µg/mL (50.0% by mass)
Example 9: 1500 µg/mL (60.0% by mass)
Example 10: 2000 µg/mL (66.7% by mass)
Example 11: 30.9 µg/mL (3.0% by mass)
Example 12: 52.6 µg/mL (5.0% by mass)
Example 13: 111.1 µg/mL (10.0% by mass)
Example 14: 250 g/mL (20.0% by mass)
Example 15: 666.7 µg/mL (40.0% by mass)
Example 16: 4000 µg/mL (80.0% by mass)
Example 17: 100.0% by mass

### <Evaluation>

### <<Odontoblast proliferation test>>

A Dulbecco's modified Eagle's medium (DMEM) added with 5% by mass of fetal bovine serum (FBS) was prepared. Then, an MDPC-23 cell (rat dental papilla-derived cell), which is a rat odontoblast-like cell line, was cultured in DMEM. Subsequently, 0.1 mL of DMEM where the MDPC-23 cells were cultured was injected into each well in a microplate (96 wells) so that 1000 MDPC-23 cells were distributed into each well.

Next, the odontoblast proliferation/differentiation promoting agents of Examples 1 to 10 and ultrapure water (control) were individually added to corresponding wells. Then, the added mixture was allowed to stand at ordinary temperature (25°C) for 5 days. Thereafter, the proliferation of the MDPC-23 cells was evaluated using a kit for measuring the number of living cells (CCK-8: Cell Counting Kit-8).

In the CCK-8 assay, a microplate reader (measurement wavelength: 450 nm) was used. The CCK-8 assay was repeated 6 times.

The results of the CCK-8 assay in Examples 1 to 5 and the control were shown in FIG. 3 and the results of the CCK-8 assay in Examples 4, 6 to 10 and the control were shown in FIG. 4.

As shown in FIGS. 3 and 4, it was found that the proliferation of the MDPC-23 cells was remarkably promoted with the odontoblast proliferation/differentiation promoting agent of each Example as compared to the control.

As shown in FIG. 4, it was found that the effect of promoting proliferation and/or differentiation of the MDPC-23 cells was gradually improved as the concentration of C-MET in the odontoblast proliferation/differentiation promoting agent was increased to 500 µg/mL (Example 7), and when the concentration of C-MET exceeded 500 µg/mL, there was not found any further improvement in the effect.

### <<Expression test of dentin formation related gene>>

A Dulbecco's modified Eagle's medium (DMEM) added with 5% by mass of fetal bovine serum (FBS) was prepared. Then, an MDPC-23 cell (rat dental papilla-derived cell) was cultured in DMEM. Subsequently, 2 mL of DMEM where the MDPC-23 cells were cultured was injected into each well in a microplate (12 wells) so that 104 MDPC-23 cells were distributed into each well.

Next, the odontoblast proliferation/differentiation promoting agents of Examples 4, 6 and 7 and ultrapure water (control) were individually added to corresponding wells. Then, the added mixture was allowed to stand at ordinary temperature (25°C) for 5 days. Thereafter, 0.02 mL of an aqueous solution of β-glycerophosphoric acid (β-GP) (10 mmol/L), 0.02 mL of an aqueous solution of ascorbic acid (50 µg/mL), and 0.002 mL of an aqueous solution of dexamethasone (100 nmol/L) (steroidal anti-inflammatory agent) were added to each well.

Then, the added mixture was allowed to stand for another 2 days and thereafter, was subjected to real-time PCR. As an internal standard, β-actin was used. The real-time PCR was repeated 3 times.

Messenger RNA (mRNA) expression levels of RNA-dentin sialophosphoprotein (rDSPP) are shown in FIG. 5A. mRNA expression levels of RNA-dentin matrix protein (rDMP-1) are shown in FIG. 5B. mRNA expression levels of RNA-osteocalcin (rOCN) are shown in FIG. 5C. mRNA expression levels of RNA-osteopontin (rOPN) are shown in FIG. 5D.

As shown in FIGS. 5A and 5B, genes that produce proteins specific to dentin (rDSPP and rDMP-1) were found to be increased, and as shown in FIGS. 5C and 5D, genes that produce bone matrix proteins (osteocalcin and osteopontin) required for hard tissues (calcification) were found to be increased. Therefore, dentin formation by the proliferated MDPC-23 cells can be expected.

### <<Measurement of cell survival rate>>

A Dulbecco's modified Eagle's medium (DMEM) added with 5% by mass of fetal bovine serum (FBS), 50 units/mL of penicillin, and 50 µg/mL of streptomycin was prepared. Then, a human dental pulp stem cell (hDPSC) was cultured in DMEM. Subsequently, 0.02 mL of β-glycerophosphoric acid (β-GP) (10 mmol/L) and 0.02 mL of ascorbic acid (50 µg/mL) were added to the DMEM for culturing hDPSC.

Subsequently, 0.1 mL of the DMEM for culturing hDPSC was injected into each well in a microplate (96 wells) so that 1,000 to 3,000 hDPSCs were distributed into each well.

Next, the odontoblast proliferation/differentiation promoting agents of Examples 9, 11 to 17 and ultrapure water (control) were individually added to corresponding wells. Then, the added mixture was allowed to stand at ordinary temperature (25°C) for 4 days. Thereafter, the cell survival rate was evaluated using a kit for measuring the number of living cells (CCK-8: Cell Counting Kit-8).

In the CCK-8 assay, a microplate reader (measurement wavelength: 450 nm) was used. The CCK-8 assay was repeated 6 times. The results were shown in FIG. 6.

As shown in FIG. 6, even though the odontoblast proliferation/differentiation promoting agent of each Example was added, the cells were found to be sufficiently viable as in the control.

### <<Differentiation test of human dental pulp stem cells into odontoblasts>>

A DMEM for culturing hDPSC was prepared in the same manner as in the measurement of cell survival rate.

The following powdery samples were each dissolved or dispersed in ultrapure water (dH₂O), and then diluted with DMEM, to thereby prepare odontoblast proliferation/differentiation promoting agents of Example 12 and Comparative Examples 1 to 4. The concentration of the sample in each odontoblast proliferation/differentiation promoting agent was 5% by mass.

### Sample:

Calcium salt of 4-MET: C-MET (Example 12),
4-methacryloxyethyltrimellitic acid: 4-MET (Comparative Example 1),
Calcium hydroxide: CH (Comparative Example 2),
Mineral trioxide aggregate: MTA (Comparative Example 3), and
Calcium chloride: CaCl₂ (Comparative Example 4).

Next, the odontoblast proliferation/differentiation promoting agents of Example 12 and Comparative Examples 1 to 4 and ultrapure water (control) were individually dissolved directly in the DMEM for culturing hDPSC.

Then, after allowed to stand for 24 days, DMEMs containing the odontoblast proliferation/differentiation promoting agents of Example 12 and Comparative Examples 1 to 4 and ultrapure water (control) dissolved therein were subjected to real-time PCR. As an internal standard, β-actin was used.

mRNA expression levels of RNA-bone sialoprotein (BSP) are shown in FIG. 7A. mRNA expression levels of RNA-osteopontin (rOPN) are shown in FIG. 7B. mRNA expression levels of RNA-osteocalcin (rOCN) are shown in FIG. 7C. mRNA expression levels of RNA-dentin sialophosphoprotein (rDSPP) are shown in FIG. 7D. mRNA expression levels of RNA-dentin matrix protein (rDMP-1) are shown in FIG. 7E.

As shown in FIGS. 7A to 7E, when C-MET was brought into contact with hDPSC, odontoblast-specific genes (BSP, rOPN, rOCB, rDSPP, and rDMP-1) were found to be increased. On the other hand, even when 4-MET, CH, and MTA were individually brought into contact with hDPSC, no increase of odontoblast-specific genes was found. Thus, it was found that C-MET promoted differentiation of hDPSC into odontoblasts.

When DMEM containing the odontoblast proliferation/differentiation promoting agent of Example 12 and ultrapure water (control) therein was allowed to stand for 14, 21, and 28 days, the calcification state thus obtained was evaluated via alkaline phosphatase (ALPase) measurement. The results were shown in FIG. 8.

Further, when DMEM containing the odontoblast proliferation/differentiation promoting agents of Example 12 and Comparative Examples 1 to 4 and ultrapure water (control) therein was allowed to stand for 30 and 32 days, the calcification state thus obtained was evaluated via alizarin red S staining measurement. The results are shown in FIGS. 9A and 9B. The evaluation of the calcification state was performed using one-way ANOVA and Tukey's HSD post-hoc test with a significance level set at 1%

As shown in FIGS. 8, 9A, and 9B, when C-MET was brought into contact with hDPSC, calcification was found to be promoted. In addition, when CH, MTA, and CaCl₂ were brought into contact with hDPSC, calcification was found. However, when CH and MTA were brought into contact with hDPSC, promotion of differentiation of hDPSC into odontoblasts was not observed as shown in FIGS. 7A to 7E. This seems that calcium ions were bonded to phosphoric acid on the cells, to thereby deposit calcium phosphate.

### <<Differentiation test of mesenchymal stem cells into odontoblasts>>

A DMEM for culturing mesenchymal stem cell (MSC) was prepared in the same manner as the measurement of cell survival rate, except that hDPSC was replaced with MSC.

Next, the odontoblast proliferation/differentiation promoting agent of Example 12 and ultrapure water (control) were individually dissolved directly in the DMEM for culturing MSC. Then, DMEM was subjected to real-time PCR when allowed to stand for 10, 17, and 24 days. As an internal standard, β-actin was used.

mRNA expression levels of RNA-bone sialoprotein (BSP) are shown in FIG. 8A. mRNA expression levels of RNA-dentin matrix protein (rDMP-1) are shown in FIG. 8B.

As shown in FIGS. 8A and 8B, when C-MET was brought into contact with MSC, odontoblast-specific genes were found to be increased. Thus, it was found that C-MET promoted differentiation of MSC into odontoblasts.

While the illustrative embodiments of the present invention are provided in the above description, such is for illustrative purpose only and it is not to be construed restrictively. Modification and variation of the present invention that will be obvious to those skilled in the art is to be covered by the following claims.

### INDUSTRIAL APPLICABILITY

The odontoblast proliferation/differentiation promoting agent of the present invention is suitably used as, for example, a pulp capping agent.

### REFERENCE SIGNS LIST

1 dental pulp
2 primary dentin
3 enamel
5 odontoblast proliferation/differentiation promoting agent
6 tertiary dentin

## Claims

1. An odontoblast proliferation/differentiation promoting agent comprising a calcium salt of 4-(meth)acryloyloxyethyltrimellitic acid.

2. The odontoblast proliferation/differentiation promoting agent according to claim 1, further comprising a bioabsorbable material that dissolves, disperses, or adsorbs the calcium salt of 4-(meth)acryloyloxyethyltrimellitic acid.

3. The odontoblast proliferation/differentiation promoting agent according to claim 1, further comprising an anti-inflammatory agent.
